# EUROPÄISCHE PATENTSCHRIFT

(11) **EP 3 152 614 B1**
(45) Veröffentlichungstag und Bekanntmachung des Hinweises auf die Patenterteilung: **21.06.2023**
(21) Anmeldenummer: 15725565.4
(22) Anmeldetag: 21.05.2015
(51) Int. Cl.: G02B 21/00, G02B 21/36, G02B 21/22, A61B 90/20

(54) **OPERATIONSMIKROSKOP MIT DATENEINHEIT UND VERFAHREN ZUM ÜBERLAGERN VON BILDERN**
OPERATING MICROSCOPE HAVING A DATA UNIT, AND METHOD FOR OVERLAYING IMAGES
MICROSCOPE OPÉRATOIRE ÉQUIPÉ D'UNE UNITÉ DE DONNÉES ET PROCÉDÉ DE SUPERPOSITION D'IMAGES

(30) Priorität: 27.05.2014 DE 102014210053
(43) Veröffentlichungstag der Anmeldung: 12.04.2017
(73) Patentinhaber: Carl Zeiss Meditec AG, 07745 Jena (DE)
(72) Erfinder: WILZBACH, Marco, 70197 Stuttgart (DE); SAUR, Stefan, 73430 Aalen (DE); PANITZ, Gerald, 73479 Ellwangen (DE)
(74) Vertreter: Carl Zeiss AG - Patentabteilung
(86) Internationale Anmeldenummer: PCT/EP2015/061262
(87) Internationale Veröffentlichungsnummer: WO 2015/181042

(56) Entgegenhaltungen:
- DE-A1- 10 243 852
- DE-A1-102009 018 633
- US-A1- 2004 070 822

## Beschreibung

### Hintergrund

Die Erfindung betrifft ein chirurgisches System mit einem Operationsmikroskop und einer Bildverarbeitungseinrichtung, welche dem vom Operationsmikroskop erzeugten Bild ein weiteres Bild überlagert.

Intraoperative Modalitäten (d.h. intraoperativ mit Sensoren gewonnene diagnostische Daten wie z.B. funktionale Hirnkartierung IOI, 5-ALA Fluoreszenz Blau400, Fluoreszenz-Tumorrand-Berechnungen, ICG Fluoreszenz IR800, Flow800, Topographie, 2D/3D Bildinformationen, ...) werden zu einem bestimmten Zeitpunkt erstellt. Manche Modalitäten können kontinuierlich (Topographiemessung), wiederholt (Blau400, Tumorrand, IR800, Flow800...), andere nur zu bestimmten Zeitpunkten während der Operation (IOI nach Öffnung der Dura, ...) erzeugt werden.

Die Aufnahme der Modalitäten erfordert meist andere Einstellungen (z.B. Beleuchtung, Filterwechsel...) oder eine Änderung im Operationsablauf. Im Normalfall sind diese Einstellungen disjunkt mit den bevorzugten Einstellungen während der Resektion von oder während eines Eingriffs im Gewebe.

Eine Lösung, die Information der verschiedenen Modalitäten in den Standardeinstellungen anzuzeigen, bietet die Augmentierung des optischen Bildes des Situs mit den Bildinformationen einer oder mehrerer der o.g. Modalitäten. Die Augmentierung kann ausgeführt sein als eine Überlagerung eines elektronisch erzeugten Bildes aus den Daten der Modalität über das optische Bild, z.B. mit Hilfe eines Teilerspiegels. Bei einer rein elektonischen Anzeige (z.B. Monitor) kann die Mischung rein digital (ohne Teilerspiegel) realisiert werden (Videosignal des Situs und der Modalität).

Ein Nachteil dabei ist, daß die Modalitäten nur zu einem bestimmten Zeitpunkt bzw. nur bei den Einstellungen, mit denen die Aufnahme gemacht wurde (Zoom, Fokus, Position (Relativposition des Operationsmikroskops zum Patienten), ...), gültig sind. Werden diese Einstellungen verändert, verlieren die Daten der Modalität ihre Gültigkeit, die Überlagerung macht keinen Sinn mehr und muß beendet werden. Manche Augmentierungen sind nur sehr kurzfristig gültig.

Sinnvoll wäre es daher, das Zeitintervall der gültigen Augmentierung zu erweitern.

Aus der US 2004/0070822 A1 ist eine Beobachtungseinrichtung mit optischen Systemen bekannt, welche die Bilder zweier Bildschirme in das Sichtfeld eines optischen Mikroskops übertragen.

Aus der DE 102 43 852 A1 ist ein Mikroskopiesystem bekannt, bei dem ein Bild abhängig von einer Schwenkstellung eines Okulartubus auf einer Anzeigevorrichtung gedreht dargestellt und in den Strahlengang des Mikroskopiesystems eingekoppelt wird.

Die DE 10 2009 018 633 A1 offenbart ein Verfahren zur intraoperativen Bildgebung von Gehirnarealen, bei dem Wärmebilder eines Gehirnareals ausgewertet und in ein mikroskopisches Bild eingespiegelt werden.

### Überblick über die Erfindung

Die vorliegende Erfindung hat die Aufgabe, die Gültigkeit der Augmentierung von intraoperativen Modalitäten zeitlich zu erweitern und robuster zu gestalten.

Diese Aufgabe wird durch das chirurgische System gemäß Anspruch 1 und durch das Verfahren gemäß Anspruch 7 gelöst.

Ein chirurgisches System gemäß einer Ausführungsform umfasst ein Operationsmikroskop mit einer Abbildungsoptik und einer Steuereinheit zum Einstellen von Abbildungsparametern des Operationsmikroskops. Hierbei kann ein stereoskopisches oder ein monoskopisches Operationsmikroskop eingesetzt werden. Weiterhin ist eine Bildverarbeitungseinrichtung zum Überlagern eines in der Bildverarbeitungseinrichtung gespeicherten Überlagerungsbildes mit einem durch das Operationsmikroskop erzeugten Bild vorgesehen, welche innerhalb eines Grundkörpers des Operationsmikroskops angeordnet sein kann. Eine Datenverarbeitungseinheit ist mit der Steuereinheit des Operationsmikroskops und mit der Bildverarbeitungseinrichtung verbunden, wobei die Datenverarbeitungseinheit mit dem Operationsmikroskop integriert sein kann oder extern bereitgestellt sein kann. Die Steuereinheit ist ausgelegt, um vor einer Veränderung wenigstens eines Abbildungsparameters des Operationsmikroskops von einem ersten Wert auf einen zweiten Wert sowohl den ersten, als auch den zweiten Wert zu speichern und der Datenverarbeitungseinheit zur Verfügung zu stellen. Dadurch wird bei jeder Umfokussierung, Positionsänderung und dergleichen sowohl der "alte", als auch der "neue" Wert des zu ändernden Abbildungsparameters erfasst und gespeichert. Die Steuereinheit ist ausgelegt, um wenigstens einen Zeitstempel zusammen mit dem ersten und/oder dem zweiten Wert zu speichern und der Datenverarbeitungseinheit zur Verfügung zu stellen. Der wenigstens eine Abbildungsparameter umfasst einen Einstellparameter von Zoom, Fokus, Lage und Ausrichtung im Raum des Operationsmikroskops und/oder des Patienten. Die Bildverarbeitungseinrichtung ist derart ausgelegt, dass sie das Überlagerungsbild entsprechend des gespeicherten ersten und zweiten Werts des wenigstens einen Abbildungsparameters modifiziert. Mittels einer geeigneten Transformationsfunktion, welche die geänderten Werte des Abbildungsparameters berücksichtigt, kann die Bildverarbeitungseinrichtung daher das Überlagerungsbild so modifizieren, dass es einer Abbildung mit dem geänderten Abbildungsparameter überlagert werden kann. Dadurch kann eine Augmentierung auch bei geänderten Abbildungsparametern erreicht werden, so dass intraoperative Modalitäten auch dann als Überlagerungsbild angezeigt werden können, wenn sich die Abbildungsparameter des Operationsmikroskops seit der Aufnahme der Modalitäten verändert hat. Die Datenverarbeitungseinheit ist ausgelegt, um wenigstens einen Grenzwert für den Wert des Abbildungsparameters bereitzustellen, wobei die Bildverarbeitungseinrichtung kein modifiziertes Überlagerungsbild erzeugt, falls der zweite Wert des Abbildungsparameters jenseits des Grenzwerts liegt.

Gemäß einiger Ausführungsformen kann die Bildverarbeitungseinrichtung ferner ausgelegt sein, um nach einer Veränderung des Abbildungsparameters von dem ersten auf den zweiten Wert das modifizierte Überlagerungsbild mit dem durch das Operationsmikroskop erzeugten Bild zu überlagern.

Ein Positionierungssystem kann vorgesehen sein, um das Operationsmikroskop an einer vorgegebenen Position im Raum zu positionieren, wobei das Positionierungssystem derart ausgelegt ist, um vor einer Veränderung eines Positionswertes des Operationsmikroskops von einem ersten Positionswert auf einen zweiten Positionswert sowohl den ersten, als auch den zweiten Positionswert zusammen mit wenigstens einem Zeitstempel zu speichern und der Datenverarbeitungseinheit zur Verfügung zu stellen.

Die Steuereinheit kann derart ausgelegt sein, dass sie erst dann den wenigstens einen Abbildungsparameter ändert, wenn das modifizierte Überlagerungsbild erzeugt worden ist, so dass das modifizierte Überlagerungsbild bereits vor der Änderung des Abbildungsparameters bereitgestellt werden kann.

Das Überlagerungsbild kann mittels wenigstens eines Strahlteilers mit dem durch das Operationsmikroskop erzeugten Bild überlagert werden.

Das Überlagerungsbild und das durch das Operationsmikroskop erzeugte Bild können jeweils als Videosignal miteinander überlagert werden.

Ein Verfahren zum Überlagern eines Überlagerungsbildes mit einem durch ein Operationsmikroskop erzeugten Bild gemäß einer Ausführungsform umfasst:
- Überlagern des Überlagerungsbildes mit dem durch das Operationsmikroskop erzeugte Bild;
- Speichern eines ersten und eines zweiten Werts wenigstens eines Abbildungsparameters des Operationsmikroskops, der einen Einstellparameter von Zoom, Fokus, Lage und Ausrichtung im Raum des Operationsmikroskops und/oder des Patienten umfasst, wobei wenigstens ein Zeitstempel zusammen mit dem ersten und/oder dem zweiten Wert gespeichert und einer Datenverarbeitungseinheit zur Verfügung gestellt wird;
- Modifizieren des Überlagerungsbildes entsprechend des gespeicherten ersten und zweiten Werts des Abbildungsparameters, wobei wenigstens ein Grenzwert für den Wert des Abbildungsparameters bereitgestellt wird und kein modifiziertes Überlagerungsbild erzeugt wird, falls der zweite Wert des Abbildungsparameters jenseits des Grenzwerts liegt;
- Verändern des Abbildungsparameters des Operationsmikroskops von dem ersten auf den zweiten Wert; und
- Überlagern des modifizierten Überlagerungsbildes mit dem durch das Operationsmikroskop erzeugte Bild.

### Detaillierte Beschreibung der Erfindung

Im folgenden werden einige Ausführungsbeispiele der Erfindung anhand der beigefügten Figuren erläutert. Es zeigt:
Fig. 1 ein chirurgisches System mit einem stereoskopischen Operationsmikroskop gemäß einer Ausführungsform der Erfindung;
Fig. 2 das chirurgische System aus Fig. 1 in einem anderen Betriebszustand;
Fig. 3 eine schematische Darstellung einer weiteren Ausführungsform eines erfindungsgemäßen chirurgischen Systems;
Fig. 4 den Workflow eines erfindungsgemäßen Verfahrens; und
Fig. 5 ein Beispiel für eine Anwendung des erfindungsgemäßen Systems und Verfahrens während einer Operation.

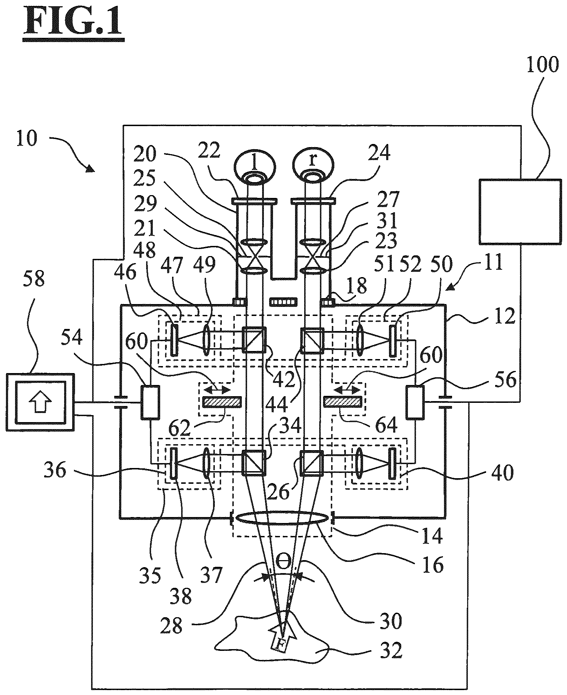

Das in Fig. 1 gezeigte chirurgische System 10 umfasst ein Operationsmikroskop 11 mit einem Operationsmikroskop-Grundkörper 12, in dem eine schaltbare Abbildungsoptik 14 mit einem Mikroskop-Hauptobjektivsystem 16 aufgenommen ist. Es weist einen an den Grundkörper 12 an einer Schnittstelle 18 angeschlossenen Binokulartubus 20 mit einem ersten und einem zweiten Okulareinblick 22, 23 für ein linkes und ein rechtes Auge 72, 74 einer Beobachtungsperson auf. Das Mikroskop-Hauptobjektivsystem 16 in dem Operationsmikroskop 11 wird von einem ersten Beobachtungsstrahlengang 28 und einem zweiten Beobachtungsstrahlengang 30 aus einem Objektbereich 32 durchsetzt.

Die Abbildungsoptik 14 enthält einen in dem ersten optischen Beobachtungsstrahlengang 28 auf der dem Objektbereich 32 abgewandten Seite des Mikroskop-Hauptobjektivsystems 16 angeordneten Auskoppel-Strahlteiler 34, der einen Teil des Beobachtungslichts aus dem ersten Beobachtungsstrahlengang 28 auskoppelt und einer Bilderfassungseinrichtung 35 zuführt. Die Bilderfassungseinrichtung 35 enthält eine erste Bilderfassungsbaugruppe 36 mit einem Objektivlinsensystem 37 und einem Bildsensor 38 sowie eine zweite Bilderfassungsbaugruppe 40 mit einem Objektivlinsensystem 37 und einem Bildsensor 38.

Darüber hinaus hat die Abbildungsoptik 14 einen in dem zweiten optischen Beobachtungsstrahlengang 30 auf der dem Objektbereich 32 abgewandten Seite des Mikroskop-Hauptobjektivsystems 16 angeordneten weiteren Auskoppel-Strahlteiler 26, der einen Teil des Beobachtungslichts aus dem zweiten Beobachtungsstrahlengang 30 auskoppelt und zu der Bilderfassungseinrichtung 35 mit dem Objektivlinsensystem 37 und dem Bildsensor 38 lenkt.

In der Abbildungsoptik 14 gibt es einen ersten Einkoppel-Strahlteiler 42 und einen zweiten Einkoppel-Strahlteiler 44. Über die Einkoppel-Strahlteiler 42, 44 kann eine an einem Display 46 einer Anzeigebaugruppe 48 einer Anzeigeeinrichtung 47 und an einem Display 50 einer Anzeigebaugruppe 52 der Anzeigeeinrichtung 47 zur Anzeige gebrachte Anzeigeinformation dem Bild des Objektbereichs 32 in dem ersten optischen Beobachtungsstrahlengang 28 und in dem zweiten optischen Beobachtungsstrahlengang 30 überlagert werden.

Mittels der Einkoppel-Strahlteiler 42, 44 kann dem Bild des Objektbereichs 32, das dem Okulareinblick 22 und dem Okulareinblick 24 des Binokulartubus 20 zugeführt wird, insbesondere ein Überlagerungsbild mit Bildinformationen aus intraoperativen Modalitäten wie eingangs beschrieben überlagert werden.

Die Anzeigeinformation 46, 50 der Anzeigebaugruppen 48, 52 wird hierfür jeweils mit einer Display-Linse 49, 51 in einen parallelen Strahlengang überführt und mittels der Tubuslinsen 21, 23 in die linke und rechte Zwischenbildebene 25, 27 des Binokulartubus 20 abgebildet. Das Zwischenbild in der linken und rechten Zwischenbildebene 25, 27 ist durch eine Okularfeldblende 29, 31 in dem Binokulartubus 20 begrenzt. Der Abbildungsmaßstab der Abbildungen der Displays 46, 50 in der linken und rechten Zwischenbildebene 25, 27 ist dabei durch das Verhältnis f_{D}/f_{T} der Brennweite f_{D} der Display-Linsen 49, 51 und der Brennweite f_{T} der Tubuslinsen 21. 23 bestimmt.

Für das Ansteuern der Displays 46, 50 enthält das chirurgische System 10 eine Bildverarbeitungseinrichtung 54 und eine Bildverarbeitungseinrichtung 56, die mit einer Datenverarbeitungseinheit 100 verbunden sind, welche innerhalb oder außerhalb des Mikroskop-Grundkörpers 12 angeordnet sein kann.

Um die mittels der Bilderfassungseinrichtungen 35, 40 aus dem ersten und dem zweiten Beobachtungsstrahlengang 28, 30 erfassten Bilder des Objektbereichs 32 darzustellen, weist das chirurgische System 10 eine vorzugsweise als 3D-Monitor ausgebildete Bildwiedergabeeinrichtung 58 auf, die mit den Bildverarbeitungseinrichtungen 54, 56 verbunden ist.

Die Abbildungsoptik 14 des Operationsmikroskops 11 enthält ein Shutterelement 62 und ein Shutterelement 64. Die Shutterelemente 62, 64 können mittels eines Antriebs (nicht gezeigt) entsprechend dem Doppelpfeil 60 verlagert werden. Mittels der Shutterelemente 62, 64 ist es möglich, den ersten und/oder den zweiten optischen Beobachtungsstrahlengang 28, 30 auf der dem Mikroskop-Hauptobjektivsystem 16 abgewandten Seite des Auskoppel-Strahlteilers 34, 26 wahlweise freizugeben oder zu sperren.

In dem in Fig. 1 gezeigten Betriebszustand des chirurgischen Systems 11 ist die Abbildungsoptik 14 so geschaltet, dass die Shutterelemente 62, 64 den ersten und den zweiten optischen Beobachtungsstrahlengang 28, 30 freigeben. Dem ersten und dem zweiten Okulareinblick 22, 24 des Binokulartubus 20 in dem Operationsmikroskop 11 wird dann der das Mikroskop-Hauptobjektivsystem 16 durchsetzende optische Beobachtungsstrahleng 28 bzw. 30 aus dem Objektbereich 32 zugeführt.

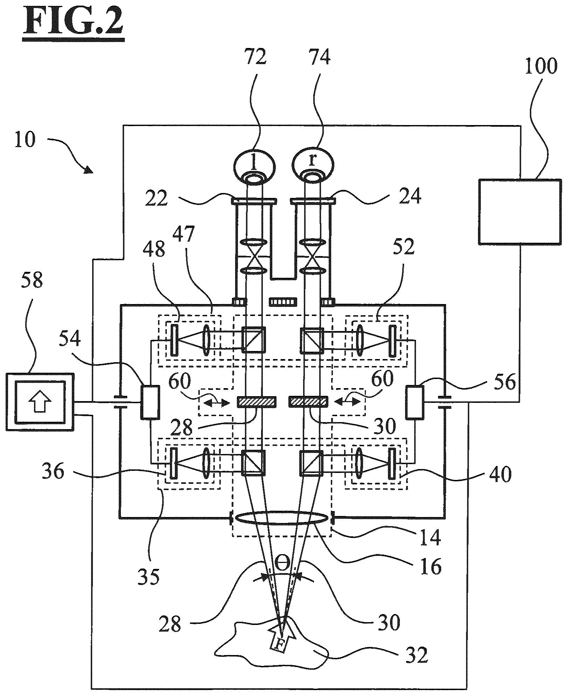

Fig. 2 zeigt das chirurgische System 10 in einem weiteren Betriebszustand, in dem die Shutterelemente 62, 64 den ersten und den zweiten optischen Beobachtungsstrahlengang 28, 30 sperren. Hier wird dem ersten und zweiten Okulareinblick 22, 24 des Binokulartubus 20 in dem Operationsmikroskop 11 jeweils das mittels der Bilderfassungsbaugruppen 36 bzw. 40 der Bilderfassungseinrichtung 35 erfasste und mit den Anzeigeeinrichtungen 48, 52 zur Anzeige gebrachte Bild des Objektbereichs 32 zugeführt.

Der Bilderfassungsbaugruppe 36 der Bilderfassungseinrichtung 35 kann also ein linkes stereoskopisches Teilbild des Objektbereichs 32 mit dem ersten optischen Beobachtungsstrahlengang 28 zugeführt werden und der Bilderfassungsbaugruppe 40 der Bilderfassungseinrichtung 35 ein rechtes stereoskopisches Teilbild des Objektbereichs 32 mit dem zweiten optischen Beobachtungsstrahlengang 30 zugeführt werden. Die optischen Achsen der beiden optischen Beobachtungsstrahlengänge 28, 30 schließen dabei einen Stereowinkel θ ein. Dies ermöglicht, mit dem Operationsmikroskop 11 auch dann den Objektbereich 32 stereoskopisch darzustellen, wenn der erste und der zweite Beobachtungsstrahlengang 28, 30 mittels der Shutterelements 62, 64 gesperrt sind. Das linke stereoskopische Teilbild in dem Binokulartubus 22 wird hierfür dann mit der Anzeigebaugruppe 48 und das rechte stereoskopische Teilbild mit der Anzeigebaugruppe 52 der Anzeigeeinrichtung 47 erzeugt.

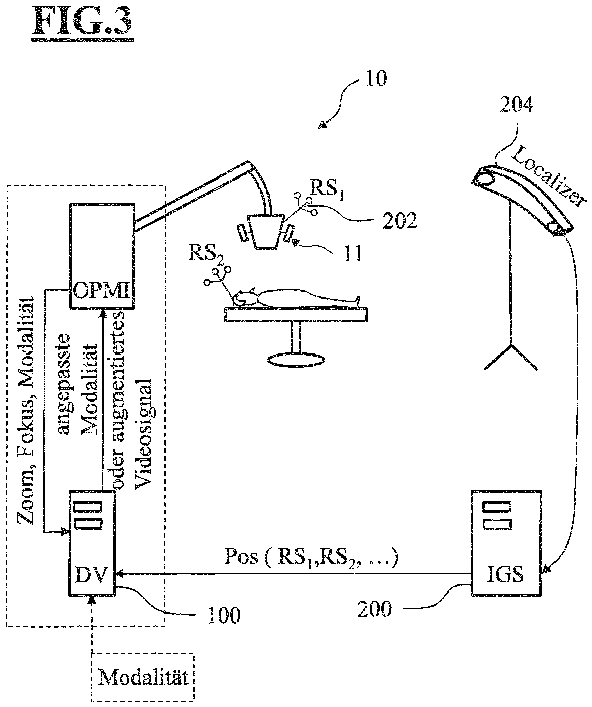

Fig. 3 zeigt ein Beispiel für ein chirurgisches System 10, wobei zusätzlich zu dem voranstehend beschriebenen Operationsmikroskop 11 und der Datenverarbeitungseinheit 100 noch ein Positionierungssystem 200 vorgesehen, welches mittels eines oder mehrerer am Operationsmikroskop 11 angebrachter Positionierungsmarker 202 und einer Positionserfassungseinheit 204 die Position und Lage im Raum des Operationsmikroskops 11 erfassen kann. Wie nachfolgend beschrieben, kann das Positionierungssystem 200 die erfassten Positionsdaten an die Datenverarbeitungseinheit 100 übertragen.

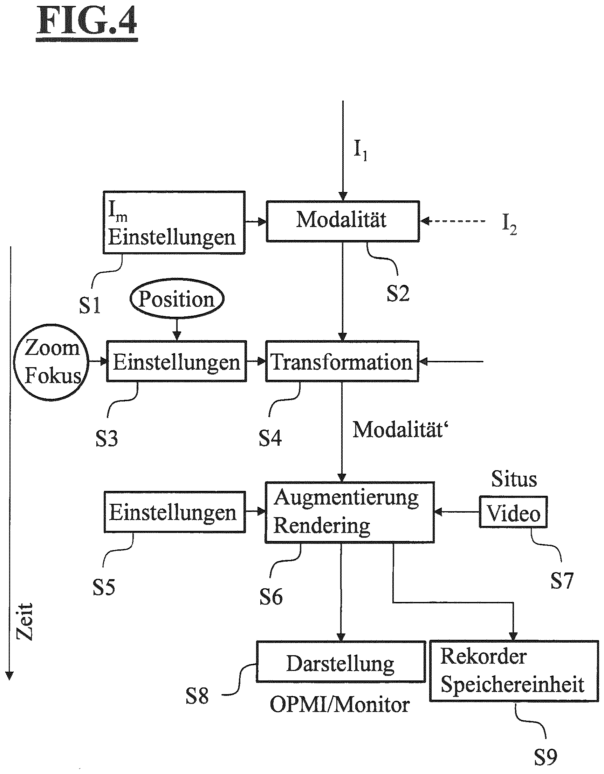

Fig. 4 zeigt ein Beispiel für ein Verfahren gemäß einer Ausführungsform. Hierbei werden jeweils die für die Augmentierung benötigten Einstellungen gespeichert, bevor eine Aktion (Änderung Position, Zoom, Fokus, Modalität, ...) am Operationsmikroskop ausgeführt wird.

Zunächst wird in Schritt 51 das Operationsmikroskop in einer ersten Einstellung betrieben und eine intraoperative Modalität wird ermittelt und gespeichert (Schritt S2). Anschließend wird eine Aktion am Operationsmikroskop getriggert, wie beispielsweise das Betätigen eines Button zum Umschalten der Modalität, Lösen der Bremsen, Ändern der Zoom- oder Fokuseinstellung, usw.

Bevor die Aktion ausgelöst wird, werden in Schritt 53 die für eine Navigation benötigten Daten von den internen Sensoren (Fokuswert, Zoomwert, Positionswerte, ...) abgefragt und mit einem Zeitstempel versehen. Sollten die Daten nicht instantan im internen Kommunikationsbus des OPMI zur Verfügung stehen, wird ein entsprechender Request mit einem eindeutigen Zeitstempel/ID verschickt, so dass eingehende Antworten dann dem Zeitstempel zugeordnet werden können. Gleichzeitig wird bei vorhandenem Positionierungssystem auch ein Request an dieses geschickt, um die Relativposition zwischen dem Operationsmikroskop und dem Patienten zu erfragen.

Diesem Request ist der gleiche Zeitstempel zugeordnet, so dass der vom Positionierungssystem bereitgestellte Wert für die Position den dazugehörigen Werten der Abbildungsparameter zugeordnet werden kann, auch wenn die Antwort vom Positionierungssystem leicht zeitverzögert eintrifft. Gleichzeitig kann auch noch ein Timeout eingebaut sein, um das Risiko einer Verzögerung zu minimieren, wenn das Positionierungssystem nicht antwortet.

Dieser Wert wird dann in Schritt S4 verwendet, um bei den nun folgenden Änderungen des Abbildungsparameters die Augmentierung entsprechend anzupassen, beispielsweise um die Größe bei Zoomänderungen anzupassen, oder um die Orientierung im Raum an eine Positionsänderung des Operationsmikroskops anzupassen.

Frühestens nachdem der Zeitstempel angelegt und das Aussenden der entsprechenden Requests angestoßen wurde, wird in Schritt S5 die ursprüngliche Aktion ausgeführt. Damit ist gewährleistet, dass der jeweils letzte gültige Wert eines Abbildungsparameters vor einer Änderung in der zentralen Datenverarbeitungseinheit gespeichert wurde. Mit der in Schritt S4 ermittelten modifizierten Modalität kann dann in Schritt S6 das Überlagerungsbild gerendert werden. Hierbei kann, wie voranstehend in Zusammenhang mit Fig. 1 beschrieben, das Überlagerungsbild mittels Strahlteiler in den Strahlengang zwischen Hauptobjektiv und Okular des Operationsmikroskops eingespiegelt werden. Alternativ kann, wie in Zusammenhang mit Fig. 2 beschrieben, das vom Hauptobjektiv erzeugte Bild als Videosignal (Schritt S7) aufgenommen und in einer Bildverarbeitungseinrichtung mit dem Überlagerungsbild, das ebenfalls als Videosignal vorliegen kann, überlagert werden.

Schließlich kann das überlagerte Bild beispielsweise an einem mit dem Operationsmikroskop verbundenen Monitor dargestellt werden (Schritt S8) oder kann aufgezeichnet und in einem Speicherelement abgespeichert werden (Schritt S9).

Die Modalitäten und die wesentlichen Einstellungen des Operationsmikroskops 11 können der zentralen Datenverarbeitungseinheit 100 zur Verfügung gestellt werden. Dann kann die Validität der Augmentierung über einen längeren Zeitraum erhalten werden. Die Modalität kann hierbei auf einer beliebigen anderen Datenverarbeitungseinheit generiert werden, also beispielsweise auch auf den Bildverarbeitungseinrichtungen 54, 56 im Operationsmikroskop 11. Es ist aber auch möglich, die Modalität auf der zentralen Datenverarbeitungseinheit 100 zu erzeugen.

Dabei spielt es vom Prinzip her keine Rolle, ob die Augmentierung rein videobasiert, oder als Überlagerung von optischem Bild und elektronisch erzeugtem Bild umgesetzt ist. Im letzteren Fall wird selbst das Videosignal des Situs nicht benötigt.

Die zentrale Datenverarbeitungseinheit 100 kann in das Operationsmikroskop 11 integriert sein, aber kann auch an beliebiger Stelle außerhalb des Operationsmikroskops 11 zur Verfügung gestellt werden. Vorteilhaft sollte jedoch gewährleistet sein, dass das anzuzeigende Videosignal bzw. die angepasste Modalität ohne wesentlichen Zeitverzug dem Arzt auf einem Anzeigegerät zur Verfügung steht. Dabei hängt die Bedeutung eines Bereitstellens "ohne wesentlichen Zeitverzug" von der jeweiligen Modalität / Applikation ab und kann sich von einigen Millisekunden bis hin zu Sekunden erstrecken.

Abbildungsparameter und Einstellungen des Operationsmikroskops sind beispielsweise Zoom, Fokus oder Positionen des Operationsmikroskops und des Patienten (wobei die Position jeweils die Lage und Ausrichtung im Raum umfassen kann, z.B. durch einen Positions- und Normalenvektor beschrieben. Ist sichergestellt, dass sich der Patient nicht bewegt, kann auch nur die Position und Ausrichtungsänderung des Operationsmikroskops zur Umrechnung verwendet werden.

Die erzeugte Modalität kann als 3D Datensatz vorliegen, wobei auch Lage und Ausrichtung im Raum durch die Positions-/Ausrichtungsmessung des Operationsmikroskops und des Patienten bekannt sind. Dieser kann nahezu für beliebige Ausrichtungen gerendert werden. Liegt die erzeugte Modalität nur als 2D bzw. 1D Datensatz vor, können neue Ausrichtungen des Operationsmikroskops 11 nur durch eine Projektion der Modalität auf die neue Ausrichtung berücksichtigt werden.

Wird eine Modalität nicht kontinuierlich erfasst, kann gemäß einiger Ausführungsformen die Gültigkeit ihrer Augmentierung von bestimmten, für jede Modalität unterschiedlichen Faktoren abhängen. Je stärker sich diese Faktoren ändern, desto größer wird die Abweichung zwischen dem Überlagerungsbild und der Realität. Gemäß einer Ausführungsform kann vorgesehen sein, die Augmentierung aufgrund der Änderung der jeweiligen Faktoren abzubrechen oder als nicht mehr unbedingt zutreffen zu markieren.

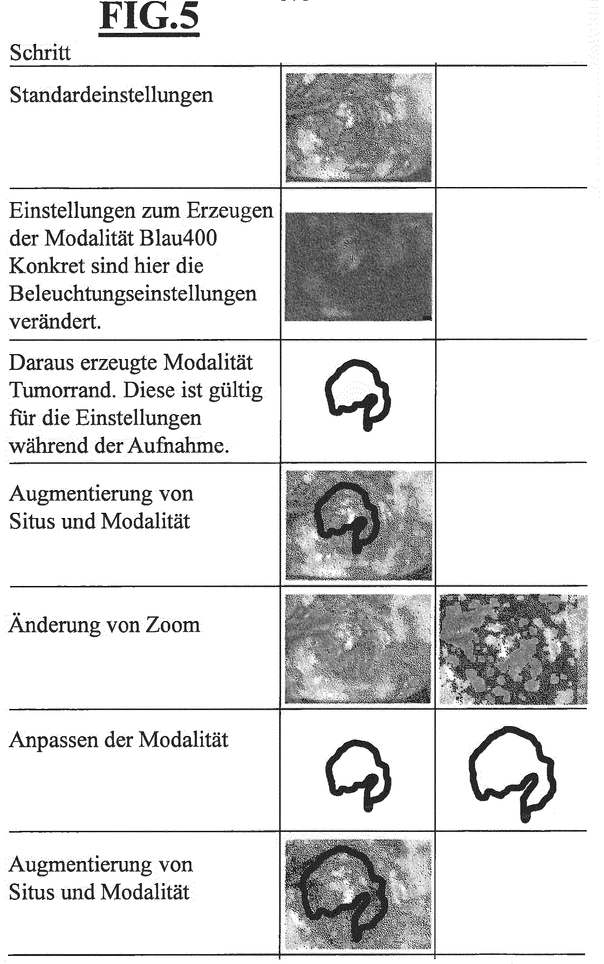

Fig. 5 zeigt ein Beispiel einer Anwendung des erfindungsgemäßen Systems und Verfahrens bei der Darstellung eines Tumorrands. Aus den Farbinformationen eines Fluoreszenzbildes wird eine Randkontur eines Tumors berechnet. Wie aus Fig. 5 und der nachstehenden Beschreibung ersichtlich wird, kann diese Kontur bei Änderungen des Zooms einfach angepasst werden. Änderungen der optischen Achse des Operationsmikroskops können nicht mehr so einfach korrigiert werden. Wird dann ein Teil des Tumors entfernt, ist die Kontur nicht mehr anpassbar.

Gemäß einer Ausführungsform können daher für eine Modalität einer oder mehrere Abbildungsparameter oder Faktoren bestimmt werden. Für diese Abbildungsparameter oder Faktoren können dann Grenzwerte für die maximale Änderung seit der letzten Bestimmung, einzeln und in Kombination, bestimmt werden. Erreicht die Änderungen des Werts des Abbildungsparameters einen dieser Grenzwerte, wird die Augmentierung ungültig. Dies kann dem Anwender durch Abbrechen der Augmentierung, durch zunehmendes Abschwächen der Augmentierung und/oder durch Darstellung eines Zuverlässigkeitsparameters als Zahl oder Symbol (Balken, Farbwechsel) mitgeteilt werden.

Die Abbildungsparameter oder Faktoren umfassen z.B. Zeit, Zoomfaktor, Bewegung der optischen Achse, Fokus (Bildebene), Verwendung von Werkzeugen (allgemein oder von bestimmten, aus dem Kamerabild erkannten Werkzeugen), Veränderung der mit Kameras erfassten chirurgischen Szene (zum Beispiel zu starke Deformation des Gewebes).

Das in Fig. 5 dargestellte Beispiel zeigt:
1. Im Blau400 Modus wird der Situs aufgenommen. Die aktuelle Position von Patient und Operationsmikroskop wird z.B. von einem externen Navigationsgerät (oder durch eine andere Lösung zur Bestimmung der Relativposition Operationsmikroskop-Patient) angefordert. Ebenfalls werden Zoom, Fokus und Beleuchtungseinstellungen gespeichert.
2. Der Tumorrand wird kontinuierlich aus den Farbinformationen des Fluoreszenzbildes berechnet. Der Tumorrand ist gültig für die jeweils gegenwärtige Ausrichtung und Fokus/Zoomeinstellung des Operationsmikroskops. Der Tumorrand liegt hier z.B. als 2D-Datensatz vor. Die Lage bzgl. Patient bestimmt sich aus den Positionen des Operationsmikroskops und des Patienten und der Fokusebene des Operationsmikroskops (Höhenunterschiede werden in diesem Beispiel nicht berücksichtigt.)
3. Es wird zurück in den Weißlichtmodus des Operationsmikroskops zurückgeschaltet. Die jeweils letzte gültige Kontur sowie die dazugehörigen Werte werden zusammengehörig (z.B. via ID oder Zeitstempel) in der zentralen Datenverarbeitungseinheit gespeichert.
4. Nun wird ein Wert eines Abbildungsparameters geändert.
   a. Der Wert für die Zoomeinstellung wird geändert: in diesem Fall liegt eine einfache Umrechnungsvorschrift vor. Der Tumorrand muß entsprechend des Zoomfaktors skaliert werden.
   b. Die Ausrichtung des Operationsmikroskops wird geändert: in diesem Fall muss die Modalität umgerechnet werden auf die neue Lage und Ausrichtung des Operationsmikroskops.

Das vorliegende chirurgische System ermöglicht es, intraoperative Modalitäten, wie beispielsweise intraoperativ mittels Sensoren gewonnene diagnostische Daten, auch dann in ein Operationsmikroskop einzuspiegeln und mit dem durch das Operationsmikroskop erzeugten Bild zu überlappen, wenn sich wenigstens Abbildungsparameter, wie beispielsweise eine Zoom-Einstellung, ein Fokuswert oder eine Position des Operationsmikroskops ändert. Durch das Abspeichern der Parameterwerte, wobei den Parameterwerten Zeitstempel zugeordnet sein können, kann weiterhin sichergestellt werden, dass zu jedem Zeitpunkt eine auch im Nachhinein dokumentierte und nachvollziehbare Umrechnungsvorschrift für die Anpassung eines Überlagerungsbilds ermittelt werden kann.

## Patentansprüche

1. Chirurgisches System (10), umfassend:
- ein Operationsmikroskop (11) mit einer Abbildungsoptik (14) und einer Steuereinheit zum Einstellen von Abbildungsparametern des Operationsmikroskops (11),
- eine Bildverarbeitungseinrichtung (54, 56) zum Überlagern eines in der Bildverarbeitungseinrichtung (54, 56) gespeicherten Überlagerungsbildes mit einem durch das Operationsmikroskop (11) erzeugten Bild, und
- eine Datenverarbeitungseinheit (100), welche mit der Steuereinheit des Operationsmikroskops (11) und mit der Bildverarbeitungseinrichtung (54, 56) verbunden ist,
wobei die Steuereinheit ausgelegt ist, um vor einer Veränderung wenigstens eines Abbildungsparameters des Operationsmikroskops (11) von einem ersten Wert auf einen zweiten Wert sowohl den ersten, als auch den zweiten Wert zu speichern und der Datenverarbeitungseinheit (100) zur Verfügung zu stellen sowie wenigstens einen Zeitstempel zusammen mit dem ersten und/oder dem zweiten Wert zu speichern und der Datenverarbeitungseinheit (100) zur Verfügung zu stellen,
wobei der wenigstens eine Abbildungsparameter einen Einstellparameter von Zoom, Fokus, Lage und Ausrichtung im Raum des Operationsmikroskops (11) und/oder des Patienten umfasst,
wobei die Bildverarbeitungseinrichtung (54, 56) derart ausgelegt ist, dass sie das Überlagerungsbild entsprechend des gespeicherten ersten und zweiten Werts des wenigstens einen Abbildungsparameters modifiziert,
und wobei die Datenverarbeitungseinheit (100) ausgelegt ist, um wenigstens einen Grenzwert für den Wert des Abbildungsparameters bereitzustellen, wobei die Bildverarbeitungseinrichtung (54, 56) kein modifiziertes Überlagerungsbild erzeugt, falls der zweite Wert des Abbildungsparameters jenseits des Grenzwerts liegt.

2. Chirurgisches System (10) nach Anspruch 1, **dadurch gekennzeichnet, dass** die Bildverarbeitungseinrichtung (54, 56) ferner ausgelegt ist, um nach einer Veränderung des Abbildungsparameters von dem ersten auf den zweiten Wert das modifizierte Überlagerungsbild mit dem durch das Operationsmikroskop (11) erzeugten Bild zu überlagern.

3. Chirurgisches System (10) nach einem der Ansprüche 1 oder 2, **dadurch gekennzeichnet, dass** ein Positionierungssystem (200) vorgesehen ist, um das Operationsmikroskop (11) an einer vorgegebenen Position im Raum zu positionieren, wobei das Positionierungssystem (200) derart ausgelegt ist, um vor einer Veränderung eines Positionswertes des Operationsmikroskops (11) von einem ersten Positionswert auf einen zweiten Positionswert sowohl den ersten, als auch den zweiten Positionswert zusammen mit wenigstens einem Zeitstempel zu speichern und der Datenverarbeitungseinheit (100) zur Verfügung zu stellen.

4. Chirurgisches System nach einem der Ansprüche 1 bis 3, **dadurch gekennzeichnet, dass** die Steuereinheit derart ausgelegt ist, dass sie erst dann den wenigstens einen Abbildungsparameter ändert, wenn aus dem gespeicherten ersten und zweiten Wert ein modifiziertes Überlagerungsbild erzeugt worden ist.

5. Chirurgisches System (10) nach einem der Ansprüche 1 bis 4, **dadurch gekennzeichnet, dass** das Überlagerungsbild mittels wenigstens eines Strahlteilers (42, 44) mit dem durch das Operationsmikroskop (11) erzeugten Bild überlagert wird.

6. Chirurgisches System (10) nach einem der Ansprüche 1 bis 4, **dadurch gekennzeichnet, dass** das Überlagerungsbild und das durch das Operationsmikroskop (11) erzeugte Bild jeweils als Videosignal miteinander überlagert werden.

7. Verfahren zum Überlagern eines Überlagerungsbildes mit einem durch ein Operationsmikroskop (11) erzeugten Bild, umfassend:
- Überlagern des Überlagerungsbildes mit dem durch das Operationsmikroskop (11) erzeugte Bild;
- Speichern eines ersten und eines zweiten Werts wenigstens eines Abbildungsparameters des Operationsmikroskops (11), der einen Einstellparameter von Zoom, Fokus, Lage und Ausrichtung im Raum des Operationsmikroskops (11) und/oder des Patienten umfasst, wobei wenigstens ein Zeitstempel zusammen mit dem ersten und/oder dem zweiten Wert gespeichert und einer Datenverarbeitungseinheit (100) zur Verfügung gestellt wird;- Modifizieren des Überlagerungsbildes entsprechend des gespeicherten ersten und zweiten Werts des Abbildungsparameters, wobei wenigstens ein Grenzwert für den Wert des Abbildungsparameters bereitgestellt wird und kein modifiziertes Überlagerungsbild erzeugt wird, falls der zweite Wert des Abbildungsparameters jenseits des Grenzwerts liegt;
- Verändern des Abbildungsparameters des Operationsmikroskops (11) von dem ersten auf den zweiten Wert; und
- Überlagern des modifizierten Überlagerungsbildes mit dem durch das Operationsmikroskop (11) erzeugte Bild.

## Claims

1. Surgical system (10), comprising:
- an operating microscope (11) with an imaging optical unit (14) and a control unit for setting imaging parameters of the operating microscope (11),
- an image processing device (54, 56) for overlaying an overlay image stored in the image processing device (54, 56) with an image generated by the operating microscope (11), and
- a data processing unit (100), which is connected to the control unit of the operating microscope (11) and to the image processing device (54, 56),
the control unit being designed such that, before a change of at least one imaging parameter of the operating microscope (11) from a first value to a second value, it stores both the first value and the second value and makes them available to the data processing unit (100), and stores at least one time stamp together with the first and/or second value and makes it available to the data processing unit (100),
the at least one imaging parameter comprising a setting parameter of the zoom, focus, position and spatial alignment of the operating microscope (11) and/or of the patient,
the image processing device (54, 56) being designed in such a way that it modifies the overlay image in a way corresponding to the stored first and second value of the at least one imaging parameter,
and the data processing unit (100) being designed such that it provides at least one limit value for the value of the imaging parameter, the image processing device (54, 56) not generating a modified overlay image if the second value of the imaging parameter is beyond the limit value.

2. Surgical system (10) according to Claim 1, **characterized in that** the image processing device (54, 56) is also designed such that, after a change of the imaging parameter from the first value to the second value, it overlays the modified overlay image with the image generated by the operating microscope (11).

3. Surgical system (10) according to either of Claims 1 and 2, **characterized in that** a positioning system (200) is provided to position the operating microscope (11) at a predetermined spatial position, the positioning system (200) being designed such that, before a change of a position value of the operating microscope (11) from a first position value to a second position value, it stores both the first position value and the second position value together with at least one time stamp and makes them available to the data processing unit (100).

4. Surgical system according to one of Claims 1 to 3, **characterized in that** the control unit is designed in such a way that it only changes the at least one imaging parameter when a modified overlay image has been generated from the stored first and second value.

5. Surgical system (10) according to one of Claims 1 to 4, **characterized in that** the overlay image is overlaid with the image generated by the operating microscope (11) by means of at least one beam splitter (42, 44).

6. Surgical system (10) according to one of Claims 1 to 4, **characterized in that** the overlay image and the image generated by the operating microscope (11) are respectively overlaid with one another as a video signal.

7. Method for overlaying an overlay image with an image generated by an operating microscope (11), comprising:
- overlaying the overlay image with the image generated by the operating microscope (11);
- storing a first value and a second value of at least one imaging parameter of the operating microscope (11) which comprises a setting parameter of the zoom, focus, position and spatial alignment of the operating microscope (11) and/or of the patient, at least one time stamp being stored together with the first and/or second value and made available to a data processing unit (100);
- modifying the overlay image in a way corresponding to the stored first and second value of the imaging parameter, at least one limit value for the value of the imaging parameter being provided and no modified overlay image being generated if the second value of the imaging parameter is beyond the limit value;
- changing the imaging parameter of the operating microscope (11) from the first value to the second value; and
- overlaying the modified overlay image with the image generated by the operating microscope (11).

## Revendications

1. Système chirurgical (10), comprenant :
- un microscope opératoire (11) pourvu d'une optique de reproduction (14) et d'une unité de commande servant à régler des paramètres de reproduction du microscope opératoire (11),
- un dispositif de traitement d'image (54, 56) servant à superposer une image de superposition stockée dans le dispositif de traitement d'image (54, 56) à une image générée par le microscope opératoire (11), et
- une unité de traitement de données (100) qui est reliée à l'unité de commande du microscope opératoire (11) et au dispositif de traitement d'image (54, 56),
dans lequel l'unité de commande est conçue, avant une modification d'au moins un paramètre de reproduction du microscope opératoire (11) d'une première valeur à une deuxième valeur, pour stocker à la fois la première et la deuxième valeur, et pour les mettre à la disposition de l'unité de traitement de données (100), ainsi que pour stocker au moins un horodatage avec la première et/ou la deuxième mémoire et pour les mettre à la disposition de l'unité de traitement de données (100),
dans lequel ledit au moins un paramètre de reproduction comprend un paramètre de réglage du zoom, du foyer, de la position et de l'orientation dans l'espace du microscope opératoire (11) et/ou du patient,
dans lequel le dispositif de traitement d'image (54, 56) est conçu de façon à modifier l'image de superposition selon la première et la deuxième valeur stockées du au moins un paramètre de reproduction,
et dans lequel l'unité de traitement de données (100) est conçue pour fournir au moins une valeur limite pour la valeur du paramètre de reproduction, dans lequel le dispositif de traitement d'image (54, 56) ne génère aucune image de superposition modifiée si la deuxième valeur du paramètre de reproduction est située au-delà de la valeur limite.

2. Système chirurgical (10) selon la revendication 1, **caractérisé en ce que** le dispositif de traitement d'image (54, 56) est en outre conçu, après une modification du paramètre de reproduction de la première à la deuxième valeur, pour superposer l'image de superposition modifiée à l'image générée par le microscope opératoire (11).

3. Système chirurgical (10) selon l'une quelconque des revendications 1 ou 2, **caractérisé en ce qu'**un système de positionnement (200) est prévu pour positionner le microscope opératoire (11) dans une position prédéfinie dans l'espace, dans lequel le système de positionnement (200) est conçu, avant une modification d'une valeur de position du microscope opératoire (11) d'une première valeur de position à une deuxième valeur de position, pour stocker à la fois la première et la deuxième valeur de position avec au moins un horodatage et pour les mettre à la disposition de l'unité de traitement de données (100).

4. Système chirurgical selon l'une quelconque des revendications 1 à 3, **caractérisé en ce que** l'unité de commande est conçue de façon à ne modifier ledit au moins un paramètre de reproduction que lorsqu'une image de superposition modifiée a été générée à partir de la première et de la deuxième valeur stockées.

5. Système chirurgical (10) selon l'une quelconque des revendications 1 à 4, **caractérisé en ce que** l'image de superposition est superposée au moyen d'au moins un séparateur de faisceaux (42, 44) à l'image générée par le microscope opératoire (11).

6. Système chirurgical (10) selon l'une quelconque des revendications 1 à 4, **caractérisé en ce que** l'image de superposition et l'image générée par le microscope opératoire (11) sont superposées l'une à l'autre respectivement comme un signal vidéo.

7. Procédé de superposition d'une image de superposition à une image générée par un microscope opératoire (11), comprenant les étapes consistant à :
- superposer l'image de superposition à l'image générée par le microscope opératoire (11) ;
- stocker une première et une deuxième valeur d'au moins un paramètre de reproduction du microscope opératoire (11) qui comprend un paramètre de réglage du zoom, du foyer, de la position et de l'orientation dans l'espace du microscope opératoire (11) et/ou du patient, dans lequel au moins un horodatage est stocké avec la première et/ou la deuxième valeur et mis à la disposition d'une unité de traitement de données (100) ;
- modifier l'image de superposition selon la première et la deuxième valeur stockées du paramètre de reproduction, dans lequel au moins une valeur limite pour la valeur du paramètre de reproduction est fournie, et aucune image de superposition modifiée n'est générée si la deuxième valeur du paramètre de reproduction est située au-delà de la valeur limite ;
- modifier le paramètre de reproduction du microscope opératoire (11) de la première à la deuxième valeur ; et
- superposer l'image de superposition modifiée à l'image générée par le microscope opératoire (11).
